# EUROPEAN PATENT SPECIFICATION

(11) **EP 1 720 610 B1**
(45) Date of publication and mention of the grant of the patent: **15.12.2010**
(21) Application number: 05726408.7
(22) Date of filing: 17.02.2005
(51) Int. Cl.: A61P 25/32, A23L 1/29, A23L 1/30, A61K 31/7004, A61K 31/194, A61K 31/375, A61K 31/195, A61K 31/198, A61K 31/525, A61K 31/122

(54) **ALCOHOL METABOLISM MODERATING COMPOSITION**
ZUSAMMENSETZUNG ZUR VERÄNDERUNG DES ALKOHOLSTOFFWECHSELS
COMPOSITION RALENTISSANT LE METABOLISME DE L'ALCOOL

(30) Priority: 17.02.2004 EP 04003529
(43) Date of publication of application: 15.11.2006
(73) Proprietor: Matuschka-Greiffenclau, Markus, Graf v., 9220 Bischofszell (CH)
(72) Inventor: Matuschka-Greinffenclau, Markus. Graf von, 9220 Bischofszell (CH); Jander, Hans Peter, 8841 Gross (CH)
(74) Representative: Dehmel, Albrecht
(86) International application number: PCT/EP2005/001644
(87) International publication number: WO 2005/077464

(56) References cited:
- EP-A- 0 185 117
- WO-A-03/006073
- FR-A- 2 748 935
- GB-A- 2 308 810
- US-A1- 2002 006 910
- PATENT ABSTRACTS OF JAPAN vol. 014, no. 334 (C-0742), 18 July 1990 (1990-07-18) & JP 02 124084 A (AJINOMOTO CO INC), 11 May 1990 (1990-05-11)

## Description

The present invention is directed to a composition, in particular a food composition, or dietary supplementation, which is active in respect to the support and/or the moderation of an alcohol degradation process within the human body.

Compositions with similar purposes have been described in, e.g., US 2002/006910; WO 03/006073; and FR 27 48935.

The present invention particularly addresses the problem of accumulation of acetaldehyde after rapid alcohol degradation i.e. alcohol metabolism as may occur in most people of Non-Caucasian type genetic structure.

In this regard, an object of the present invention is to achieve solutions which provide a reduction in the toxic effects or physiological stress in connection with the consumption of alcohol, in particular for people with a predisposition towards rapid alcohol degradation and subsequent accumulation of acetaldehyde due to a genetic polymorphism of human acetaldehyde-dehydrogenase enzym.

According to the present invention this object is attained by a food composition, or dietary supplementation including the following substances:
dextrose, Vitamin C, L-glutamine cysteine, riboflavin, succinic acid, fumaric acid, and
coenzyme Q 10,
all substances in physiologically relevant doses.

With this particular food composition, or dietary supplementation it will become possible to reduce the activity, or to suppress the production of a particular alcohol dehydrogenase enzyme (ADH₃) and to slow down the production of acetaldehyde and the ethanol metabolism process. Further the enzymatic activity of aldehyde dehydrogenase ALDH₂ will be enhanced, so that the metabolisation of acetaldehyde will be supported.

These particular effects help to reduce a flushing syndrome, reduce the likelihood of headaches and also help to avoid or ease a hangover the day after.

The particular food composition or dietary supplementation is considered to be appropriate to reduce a peak of excess acetaldehyde entering the blood stream and is intended to lower the risk of damage to vital organs and functions of the human body, and in this connection also lower the risk of several forms of cancer.

Preferably this food composition or supplements should be taken about 5 minutes prior to consumption of alcohol and in case of high alcohol consumption again whilst consuming alcohol. The mass of the food composition taken by the consumer should be in the range of about 70 to 120% of the mass of the alcohol included in the consumed drinks. A standard dose might include about 10.0g dextrose, 1.0g Vitamin C, 1.5g L-glutamine, 500mg cysteine, 40mg riboflavin, 100 mg succinic acid, 100 mg fumaric acid and 60mg coenzyme Q10.

The particular food composition, or dietary supplementation is intended to prevent too much acetaldehyde passing into the mitochondrial matrix and to suppress self-blockade of the enzymatic activity of ALDH and thus facilitate its the decomposition of acetaldehyde.

The physiological risks in connection with alcohol consumption may therefore be significantly reduced by the use of the food composition according to the present invention, as this food composition or dietary supplementation facilitates in a synergetic manner an early decrease of the level of acetaldehyde after drinking and simultaneously provides a protective effect in respect of the suppression of the generation of free radicals.

Said food composition or dietary supplementation is preferably in such a form, preferably as ingredients of a kind of aperitif, that it allows the food composition to be consumed within a restaurant or a bar prior to consuming alcoholic drinks. Preferably a dosage for a person with a body weight of about 80 kg includes a dextrose fraction of approx. 75%, wherein the said dosage may have an overall weight of about 10 to 15, preferably 13.3g. Such a dosage is to provide a considerable moderation in degrading about 18ml alcohol.

The food composition or dietary supplementation is preferably constituted in a manner wherein a dosage of same, includes a dextrose fraction of about 75.2 mass%, i.e. a quantity of dextrose in the range from 7.2 to 12.8g, preferably 10.0 g within a dose of 13,3g.

The food composition or dietary supplementation is preferably constituted in a manner wherein a dosage of same includes a Vitamin C fraction of about 7.5 mass% i.e a quantity of Vitamin C in the range from 0.78 to 1.18 g, preferably 1.0g, within a dose of 13.3g.

The food composition or dietary supplementation is preferably constituted in a manner wherein a dosage of same includes a L-glutamine fraction of about 11.27 mass%, i.e. a quantity of said L-glutamine fraction in the range from 1.23 to 1.7 g, preferably 1.5g, within a dose of 13.3g.

The food composition or dietary supplementation is preferably constituted in a manner wherein a dosage of same includes a cysteine fraction of about 3.76 mass%, i.e. a quantity of said cysteine fraction in the range from 460 to 540 mg, preferably 500mg, within a dose of 13.3g.

The food composition or dietary supplementation is preferably constituted in a manner wherein a dosage of same includes a riboflavin fraction of about 0.30 mass% i.e. a quantity of said riboflavin in the range from 32 to 48 mg, preferably 40mg, within a dose of 13.3g.

The food composition or dietary supplementation is preferably constituted in a manner wherein a dosage of same includes a succinic acid (Bernsteinsäure) fraction of about 0.752 mass%, i.e. a quantity of said succinic acid in the range from 90 to 110 mg, preferably 100mg, within a dose of 133g.

The food composition or dietary supplementation is preferably constituted in a manner wherein a dosage of same includes a fumaric acid (Fumarsäure) fraction of about 0.752 mass%, i.e. a quantity of said fumaric acid in the range from 90 to 110 mg, preferably 100mg, within a dose of 13.3g.

The food composition or dietary supplementation is preferably constituted in a manner wherein a dosage of same includes a coenzyme fraction of about 0,451 mass%, i.e. a quantity of said coenzyme fraction in the range from 50 to 70 mg, preferably 60mg, within a dose of 13.3g.

The food composition or dietary supplementation is preferably constituted in a manner wherein a dosage of same is in the form of tablets. Preferably, each tablet is so shaped and dimensioned that it allows said tablet to be easily swallowed.

Preferably, said tablets are in such a form that one dosage includes a plurality of those tablets.

The tablets may be accommodated within a dosage receptacle which includes a number of those tablets. It is possible for the food composition to be in the form of small tablets or balls, and to keep same in a small tube, while the volume of the food composition taken by the consumer can be determined with respect to the volume of alcohol which is expected to be consumed.

The food composition or dietary supplementation may also be in a form similar to sugar-cubes, or might be in the form of cryopowder.

The food composition or dietary supplementation may be separated into separate subunits. It is possible to provide one unit, for example a capsule including the Vitamin C fraction, cysteine, riboflavin, succinic acid, fumaric acid and coenzyme Q10, whilst most of the dextrose fraction is kept in separate units, capsules, tablets or the like.

It is possible to add further substances such as fruit juice extracts, curcuma, tannin, a powder of Panax notoginseng, and Vinca rosea in suitable amounts. Oolong tea, aloe vera and spiral water algae might also be added.

The food-composition or dietary supplementation may also be in the form of a liquid, in particular a sirup-type liquid. It is possible to provide the food composition in the appearance of a soft drink in a small bottle.

The particular food composition or dietary supplementation is considered to provide the following achievements:
1. Reduction of ethanol metabolism by slowing down the process of ethanol oxidation into acetaldehyde, to prevent accumulation of acetaldehyde in the first place.
2. Stimulation of the activity of ALDH and avoiding any blockade of its enzymatic activity.
3. Speeding up the reaction from acetaldehyde to acetic acid and the further decomposition in the citrate cycle.
4. Improving the levels of those anti-oxidants of the alcohol consumer, which specially protect against toxic effects of acetaldehyde.

### The first achievement

is believed to be reached by the intake of a large dose of dextrose sugar (glucose). Glucose is rapidly oxidised in the cytosol of liver cells using the same cytosol NAD+ pool used by ethanol to be converted into acetaldehyde. Because the amount of cytosolic NAD+ is limited and can only constantly be reproduced from NADH+H much less acetaldehyde accumulates.

### The second achievement

is also believed to be achieved by the intake of a large dose of glucose. Glucose augments the enzymatic activity of ADH as well as of ALDH. When a large glucose load occurs in the cytosol of liver cells then there is no possibility that the acetaldehyde reaches levels which could lead to inactivation of ALDH or to mitochondrial destruction.

### The third achievement

is believed to be performed by
a) Accelerating the reoxidation from NADH+H+ to NAD+ by speeding up the transport of electrons through the inner mitochondrial membrane
b) Accelerating the Krebs cycle

It is believed to be achieved by the inclusion of coenzyme Q₁₀ and riboflavin. Riboflavin will quickly be transformed to FMN, which together with coenzyme Q₁₀ is the determining substance for the speed of the reoxidation of NADH+H⁺ to NAD⁺ in the mitochondrial matrix. Acetaldehyde needs NAD+ when it is metabolised to acetic acid. Within this reaction NAD⁺ is transformed into NADH+H⁺. Because the availability of NAD⁺ is limited in the mitochondrial matrix NADH+H⁺ has to be re-transformed into NAD⁺ to serve again for acetaldehyde decomposition. This reaction is only possible because FMN and coenzyme Q₁₀ absorb the electrons of NADH+H⁺ and shuttle them through the mitochondrial membrane. The more FMN and coenzyme Q₁₀ are available, the more this process is speeded up and, because more NAD⁺ is available, the metabolism of acetaldehyde is accelerated.

The inclusion of coenzyme Q₁₀ also makes also sense because its level decreases in the human body with progressing age.

The activation of the Krebs (citrate) cycle is believed to be achieved by the inclusion of succinic acid and fumaric acid. Both substances activate the second half of the citrate cycle and thereby activate the aerobic oxidation process in mitochondria. L-glutamine helps to speed up the mitochondria-cytosolic malate-asparate shuttle, which plays a key role in the course of intoxication by acetaldehyde. It also speeds up the succinate oxidation process by preventing oxalic and acetic inhibition of succinate dehydrogenase.

### The fourth achievement,

the elevation of anti-oxidant levels, is believed to be achieved by the inclusion of cysteine, ascorbic acid and also of L-glutamine. Cysteine should provide a strong anti-oxidant effect as well as ascorbic acid. The human body transforms cysteine to gluthatione which specially protects against the toxic effects of acetaldehyde. To reach an optimal level of gluthatione and to avoid cysteine being transformed to cystine, it is important to combine cysteine with glutamine and give twice as much ascorbic acid as cysteine.

By taking the mentioned substances, it is expected that the level of acetaldehyde after drinking alcohol will be remarkably reduced and flushing symptoms at least diminished. The other known side-effects of acetaldehyde such as headaches and hangovers should also disappear.

## Claims

1. Food composition, or dietary supplementation for moderating an alcohol degradation process in respect to ethanol metabolism within the human body, including the following substances in physiologically relevant amount:
dextrose,
Vitamin C,
L-glutamine,
cysteine,
riboflavin,
succinic acid, and/or fumaric acid,
coenzyme Q10.

2. Food composition or dietary supplementation according to claim 1, wherein a dose of same has a weight of about 13.3g, said dose being configured in a manner which allows same to be consumed within a restaurant or a bar prior to the consumption of alcohol.

3. Food composition or dietary supplementation according to claim 1 or 2, wherein a dose of same includes a dextrose fraction of about 75.2 mass%.

4. Food composition or dietary supplementation according to any one of claims 1 to 3, wherein a dose of same includes a Vitamin C fraction of about 7.5 mass%.

5. Food composition or dietary supplementation according to any one of claims 1 to 4, wherein a dose of same includes a L-glutamine fraction of about 11.28 mass%.

6. Food composition or dietary supplementation according to any one of claims 1 to 5, wherein a dose of same includes a cysteine fraction of about 3.76 mass%.

7. Food composition or dietary supplementation according to any one of claims I to 6, wherein a dose of same includes a riboflavin fraction of about 0.3 mass%.

8. Food composition or dietary supplementation according to any one of claims 1 to 7, wherein a dose of same includes a succinic acid fraction of about 0.752 mass%.

9. Food composition or dietary supplementation according to any one of claims I to 8, wherein a dose of same includes a fumaric acid fraction of about 0.752 mass%.

10. Food composition or dietary supplementation according to any one of claims 1 to 9, wherein a dose of same includes a coenzyme fraction of about 0.451 mass%.

11. Food composition or dietary supplementation according to any one of claims 1 to 10, wherein same is in the form of tablets.

12. Food composition or dietary supplementation according to any one of claims 1 to 10, wherein a dose of same includes a plurality of small tablets or capsules.

13. Food composition or dietary supplementation according to any one of claims I to 10, wherein said tablets or capsules are contained in a dosage receptacle.

14. Food composition or dietary supplementation according to any one of claims 1 to 10, wherein said composition is of a sugar-cube type form.

15. Food composition or dietary supplementation according to any one of claims I to 10, wherein same is in the form of cryopowder.

16. Food composition or dietary supplementation according to any one of claims I to 10, wherein same is in the form of a small drink unit.

17. Food composition or dietary supplementation according to any one of claims 1 to 10, wherein same is in the form of sirup.

18. Food composition, or dietary supplementation for affecting alcohol degrading process in respect to ethanol metabolism within the human body, according to any one of claims 1 to 17, for providing the following effects within the human body:
- Reducing ethanol metabolism by slowing down the process of ethanol oxidation into acetaldehyde, to prevent accumulation of acetaldehyde in the first place;
- Stimulating the activity of ALDH and avoiding any blockade of its enzymatic activity;
- Speeding up the reaction from acetaldehyde to acetic acid and further decomposition in the citrate cycle;
- Improving the levels of those anti-oxidants of the alcohol consumer which specially protect against toxic effects of acetaldehyde.

## Patentansprüche

1. Nahrungsmittelzusammensetzung oder Nahrungsergänzung zur Beeinflussung eines Alltolabbauprozesses im Hinblick auf den Ethanol-Metabolismus im menschlichen Körper, das die folgenden Substanzen in physiologisch relevanter Menge einschließt:
Dextrose,
Vitamin C,
L-Glutamin,
Cystein,
Riboflavin,
Succinylsäure, und/oder Fumarsäure,
Coenzym Q10.

2. Nahrungsmittelzusammensetzung oder Nahrungsergänzung nach Anspruch 1, wobei eine Dosis derselben ein Gewicht von ungefähr 13,3 g hat, wobei diese Dosis in der Art konfiguriert ist, dass dieselbe in einem Restaurant oder einer Bar vor dem Konsumieren von Alkohol verzehrt werden kann.

3. Nahrungsmittelzusammensetzung oder Nahrungsergänzung nach Anspruch 1 oder 2, wobei eine Dosis derselben einen Dextrose-Anteil von ungefähr 75,2 Massenprozent einschließt.

4. Nahrungsmittelzusammensetzung oder Nahrungsergänzung nach einem der Ansprüche 1 bis 3, wobei eine Dosis derselben einen Vitamin C-Anteil von ungefähr 7,5 Massenprozent einschließt.

5. Nahrungsmittelzusammensetzung oder Nahrungsergänzung nach einem der Ansprüche 1 bis 4, wobei eine Dosis derselben einen L-Glutamin-Anteil von ungefähr 11,28 Massenprozent einschließt.

6. Nahrungsmittelzusammensetzung oder Nahrungsergänzung nach einem der Ansprüche 1 bis 5, wobei eine Dosis derselben einen Cystein-Anteil von ungefähr 3,76 Massenprozent einschließt.

7. Nahrungsmittelzusammensetzung oder Nahrungsergänzung nach einem der Ansprüche 1 bis 6, wobei eine Dosis derselben einen Riboflavin-Anteil von ungefähr 0,3 Massenprozent einschließt.

8. Nahrungsmittelzusammensetzung oder Nahrungsergänzung nach einem der Ansprüche 1 bis 7, wobei eine Dosis derselben einen Succinylsäure-Anteil von ungefähr 0,752 Massenprozent einschließt.

9. Nahrungsmittelzusammensetzung oder Nahrungsergänzung nach einem der Ansprüche 1 bis 8, wobei eine Dosis derselben einen Fumarsäure-Anteil von ungefähr 0,752 Massenprozent einschließt.

10. Nahrungsmittelzusammensetzung oder Nahrungsergänzung nach einem der Ansprüche 1 bis 9, wobei eine Dosis derselben einen Coenzym-Anteil von ungefähr 0,451 Massenprozent einschließt.

11. Nahrungsmittelzusammensetzung oder Nahrungsergänzung nach einem der Ansprüche 1 bis 10, wobei dieselbe in der Form von Tabletten vorliegt.

12. Nahrungsmittelzusammensetzung oder Nahrungsergänzung nach einem der Ansprüche 1 bis 10, wobei eine Dosis derselben eine Vielzahl an kleinen Tabletten oder Kapseln einschließt.

13. Nahrungsmittelzusammensetzung oder Nahrungsergänzung nach einem der Ansprüche 1 bis 10, wobei diese Tabletten oder Kapseln in einem Dosierungseinheitsbehälter enthalten sind.

14. Nahrungsmittelzusammensetzung oder Nahrungsergänzung nach einem der Ansprüche 1 bis 10, wobei diese Zusammensetzung eine Art Würfelzucker-Form aufweist.

15. Nahrungsmittelzusammensetzung oder Nahrungsergänzung nach einem der Ansprüche 1 bis 10, wobei dieselbe in der Form von Kryopulver vorliegt.

16. Nahrungsmittelzusammensetzung oder Nahrungsergänzung nach einem der Ansprüche 1 bis 10, wobei dieselbe in der Form einer kleinen Trinkeinheit vorliegt.

17. Nahrungsmittelzusammensetzung oder Nahrungsergänzung nach einem der Ansprüche 1 bis 10, wobei dieselbe in der Form eines Sirups vorliegt.

18. Nahrungsmittelzusammensetzung oder Nahrungsergänzung zur Beeinflussung des Alkoholabbauprozesses im Hinblick auf den Ethanol-Metabolismus im menschlichen Körper nach einem der Ansprüche 1 bis 17 zur Bereitstellung der folgenden Wirkungen im menschlichen Körper:
- Reduzieren des Ethanol-Metabolismus durch Verlangsamen des Ethanol-Oxidationsprozess zu Acetaldehyd um in erster Linie die Akktunulation von Acetaldehyd zu verhindern,
- Stimulieren der Aktivität von ALDH und Vermeiden eines Blockierens deren enzymatischer Aktivität,
- Beschleunigen der Reaktion von Acetaldehyd zu Essigsäure und weitere Zersetzung im Citrat-Zyklus,
- Verbessern der Konzentrationen der Antioxidantien des Alkoholkonsumierenden, die speziell gegen toxische Wirkungen von Acetaldehyd schützen.

## Revendications

1. Composition alimentaire ou complément alimentaire pour modérer un processus de dégradation de l'alcool par rapport au métabolisme de l'éthanol au sein du corps humain, comprenant les substances suivantes dans une quantité physiologiquement pertinente :
dextrose,
vitamine C,
L-glutamine,
cystéine,
riboflavine,
avide succinique et/ou acide fumarique,
coenzyme Q10.

2. Composition alimentaire ou complément alimentaire selon la revendication 1, où une dose de celle-ci ou de celui-ci a un poids d'environ 13,3 g, ladite dose étant configurée d'une manière qui permet la consommation de celle-ci ou de celui-ci dans un restaurant ou un bar avant la consommation d'alcool.

3. Composition alimentaire ou complément alimentaire selon la revendication 1 ou 2, où une dose de celle-ci ou de celui-ci comprend une fraction de dextrose d'environ 75,2 % en masse.

4. Composition alimentaire ou complément alimentaire selon l'une quelconque des revendications 1 à 3, où une dose de celle-ci ou de celui-ci comprend une fraction de vitamine C d'environ 7,5 % en masse.

5. Composition alimentaire ou complément alimentaire selon l'une quelconque des revendications 1 à 4, où une dose de celle-ci ou de celui-ci comprend une fraction de L-glutamine d'environ 11,28 % en masse.

6. Composition alimentaire ou complément alimentaire selon l'une quelconque des revendications 1 à 5, où une dose de celle-ci ou de celui-ci comprend une fraction de cystéine d'environ 3,76 % en masse.

7. Composition alimentaire ou complément alimentaire selon l'une quelconque des revendications 1 à 6, où une dose de celle-ci ou de celui-ci comprend une fraction de riboflavine d'environ 0,3 % en masse.

8. Composition alimentaire ou complément alimentaire selon l'une quelconque des revendications 1 à 7, où une dose de celle-ci ou de celui-ci comprend une fraction d'acide succinique d'environ 0,752 % en masse.

9. Composition alimentaire ou complément alimentaire selon l'une quelconque des revendications 1 à 8, où une dose de celle-ci ou de celui-ci comprend une fraction d'acide fumarique d'environ 0,752 % en masse.

10. Composition alimentaire ou complément alimentaire selon l'une quelconque des revendications 1 à 9, où une dose de celle-ci ou de celui-ci comprend une fraction de coenzyme d'environ 0,451 % en masse.

11. Composition alimentaire ou complément alimentaire selon l'une quelconque des revendications 1 à 10, où celle-ci ou celui-ci est sous la forme de comprimés.

12. Composition alimentaire ou complément alimentaire selon l'une quelconque des revendications 1 à 10, où une dose de celle-ci ou de celui-ci comprend une pluralité de petits comprimés ou de capsules.

13. Composition alimentaire ou complément alimentaire selon l'une quelconque des revendications 1 à 10, où lesdits comprimés ou lesdites capsules sont contenus dans un réceptacle posologique.

14. Composition alimentaire ou complément alimentaire selon l'une quelconque des revendications 1 à 10, où ladite composition est d'une forme type cube de sucre.

15. Composition alimentaire ou complément alimentaire selon l'une quelconque des revendications 1 à 10, où celle-ci ou celui-ci est sous la forme de cryopoudre.

16. Composition alimentaire ou complément alimentaire selon l'une quelconque des revendications 1 à 10, où celle-ci ou celui-ci est sous la forme d'une petite unité de boisson.

17. Composition alimentaire ou complément alimentaire selon l'une quelconque des revendications 1 à 10, où celle-ci ou celui-ci est sous la forme d'un sirop.

18. Composition alimentaire ou complément alimentaire destiné à affecter le processus de dégradation de l'alcool par rapport au métabolisme de l'éthanol au sein du corps humain selon l'une quelconque des revendications 1 à 17, pour procurer les effets suivants au sein du corps humain :
- la réduction du métabolisme de l'éthanol en ralentissant le processus d'oxydation de l'éthanol en acétaldéhyde, pour empêcher l'accumulation d'acétaldéhyde en premier lieu ;
- la stimulation de l'activité de l'ALDH et l'évitement de tout blocage de son activité enzymatique ;
- l'accélération de la réaction de l'acétaldéhyde en acide acétique et en outre de la décomposition dans le cycle des citrates ;
- l'amélioration des taux des antioxydants du consommateur d'alcool qui protègent spécialement contre les effets toxiques de l'acétaldéhyde.
